# EUROPEAN PATENT SPECIFICATION

(11) **EP 0 465 910 B1**
(45) Date of publication and mention of the grant of the patent: **08.11.1995**
(21) Application number: 91110405.7
(22) Date of filing: 24.06.1991
(51) Int. Cl.: A61B 17/04

(54) **Improved suture anchor and driver assembly**
Nähfadenanker und Installierwerkzeug
Ancrage de fil de suture et outil d'installation

(30) Priority: 13.07.1990 US 552440; 05.04.1991 US 681042
(43) Date of publication of application: 15.01.1992
(73) Proprietor: AMERICAN CYANAMID COMPANY, Wayne, NJ 07470-8426 (US)
(72) Inventor: Cerier, Jeffrey C., Franklin, Massachusetts 02038 (US); Warren, Russell F., Greenwich, Connecticut 06831 (US); Carlozzi, Gerald S., Weymouth, Massachusetts 02190 (US); DiCarlo, Paul, East Falmouth, Massachusetts 02536 (US); Dwyer, James W., South Brookfield, Connecticut 06804 (US)
(74) Representative: Wächtershäuser, Günter, Prof. Dr.

(56) References cited:
- EP-A- 0 241 240
- EP-A- 0 361 756
- EP-A- 0 464 479
- WO-A-89/01767
- WO-A-89/01767
- WO-A-89/10096
- US-A- 4 632 100
- US-A- 4 632 100
- US-A- 4 640 271
- US-A- 4 870 957
- US-A- 4 946 468
- US-A- 4 946 468

## Description

This is a continuation-in-part of common-owned application Serial No. 552,440, filed on July 13, 1990.

### TECHNICAL FIELD

A suture anchor for implanting in bone or tissue for surgical use is disclosed. The anchor is securely affixed on the end of a driver device and positioned and installed during surgery.

### BACKGROUND ART

Anchoring or affixation devices and systems for medical or surgical use are in common use today. The anchors are implanted to aid in holding bone, tissue, ligaments and the like together or in place until healing takes place, or are used temporarily to help perform a procedure necessary for optimum surgical results. When implanted permanently, the anchors can be made from an absorbable material if desired.

Bone and suture anchors are used, for example, during orthopedic surgery to hold fractured bones together, affix ligaments to bones and to aid in determining isometric placement of anterior cruciate ligament replacements. Examples of such anchors are shown in U.S. Patent Nos. 4,537,185, 4,632,100, 4,640,271, 4,723,541 and 4,738,255.

Some positioning and driver mechanisms and systems are known which are used to accurately position and install the bone anchors in place. Examples of such mechanisms and systems are disclosed, for example, in U.S. Patent Nos. 3,990,438, 4,537,185, 4,632,100, 4,723,541 and 4,738,255. Two other known systems are the "Statak" mechanism marketed by Zimmer, Inc., Warsaw, Indiana and the "Isotac" mechanism marketed by Acufex Microsurgical, Inc., Norwood, Massachusetts.

An apparatus and method for manipulating and anchoring tissue is disclosed in W089/10096. The apparatus includes an anchor member with attached suture. The anchor member is lodged within the tip of a needle which is inserted into a joint. A tube fits within the needle and is pushed towards the needle tip to expell the anchor member into or behind the tissue to manipulated or anchored.

In prior, non-prepublished European patent application EP-A-0 464 479, the present applicant has described a bone anchor and method of anchoring a suture to a bone. The bone anchor is installed in the bone by means of an inserter device and a spreader device.

It is an object of the present invention to provide an improved bone or suture anchor for surgical use. It is another object of the present invention to provide an improved driver device for a bone or suture anchor.

It is a still further object of the invention to provide a surgical anchor and driver assembly which allows efficient and accurate positioning and placement of a bone or suture anchor during surgery, the anchor being able to be affixed either for permanent or temporary use, and the driver being able to remove the installed anchor if desired.

It is also an object of the invention to provide an anchor and driver assembly in which the bone or suture anchor can be temporary securely affixed to the driver device for installation and removal, and be removed easily from the driver device when desired. It is another object to provide means on the driver device to hold the anchor temporarily in place and prevent it from rotating relative to the driver device.

It is still another object to provide a surgical bone or suture anchor and driver assembly in which sutures holding the anchor in place are recessed or positioned in a way not to obstruct or cause a problem during positioning, installation and/or removal of the anchor.

Another object of the invention is to provide a guide member for determining and limiting the depth of insertion of the anchor in the bone.

These and other objects are met by the present invention which will become apparent upon review of the following detailed description of the invention, when taken by itself or in view of the drawings.

The object of the present invention is solved with a device for anchoring a suture to a bone as defined in Claim 1.

### DISCLOSURE OF INVENTION

The present invention relates to a unique and improved anchor, an anchor driver device, and a combined anchor and driver assembly. The anchor is a bone or suture anchor with a rounded, conical or pointed tip and one or more ridges around its circumference which are used to help install (force fit) the anchor into a previously drilled hole in bone or tissue. The ridges act to hold the anchor in position in the hole for its intended use. A suture is positioned through a hole in the anchor and extends from the anchor for subsequent use by the surgeon (for example, to hold ligaments, bones or tissue in place, or for use during isometric testing of the positioning for a substitute anterior cruciate ligament).

The anchor is adapted to be securely positioned on the end of a driver device. The driver has a handle and elongated shaft. The anchor snaps or force fits onto the end of the shaft and is keyed against rotation. A guide member can be used to ensure correct depth of placement of the anchor and protect the sutures during use. The suture is affixed to the handle of the driver and grooves or slots are provided along the driver shaft for positioning of the sutures during use of the anchor and driver assembly. By positioning the sutures in the grooves, they are placed out of the way so they cannot interfere with positioning, placement, installation or removal of the anchor.

In an alternative embodiment, the anchor is hollow and the driver handle and shaft are cannulated. This allows the sutures to be passed through the anchor and driver and not interfere with the installation or removal procedures.

### BRIEF DESCRIPTION OF DRAWINGS

FIGURE 1 illustrates the unique anchor and driver assembly with the anchor being positioned and held in place on the driver;
FIGURE 2 is a partial exploded view of the anchor and the end of the driver illustrating their features and showing how they are affixed together;
FIGURE 3 is a partial exploded view similar to Figure 2 but with the anchor and driver shaft rotated 90° relative to the view shown in Figure 2 and with the suture removed for ease of viewing;
FIGURE 4 is a cross-sectional view of the driver shaft taken along lines 4-4 in Figure 3;
FIGURE 5 is an end plan view of the anchor viewed in the direction of the arrows 5-5 in Figure 3;
FIGURE 6 is an end plan view of the driver device viewed in the direction of the arrows 6-6 in Figure 1;
FIGURE 7 is a cross-sectional view of the anchor taken along lines 7-7 in Figure 3;
FIGURES 8 and 9 illustrate alternate embodiments of an anchor and driver device;
FIGURES 10, 10A and 11 illustrate the use of the inventive anchor and driver assembly with a depth guide member;
FIGURE 12 illustrates the "setting" of the anchor;
FIGURE 13 illustrates the use of the invention to affix soft tissue to a bone; and
FIGURE 14 depicts another embodiment of the rod-type anchor.

### BEST MODE FOR CARRYING OUT THE INVENTION

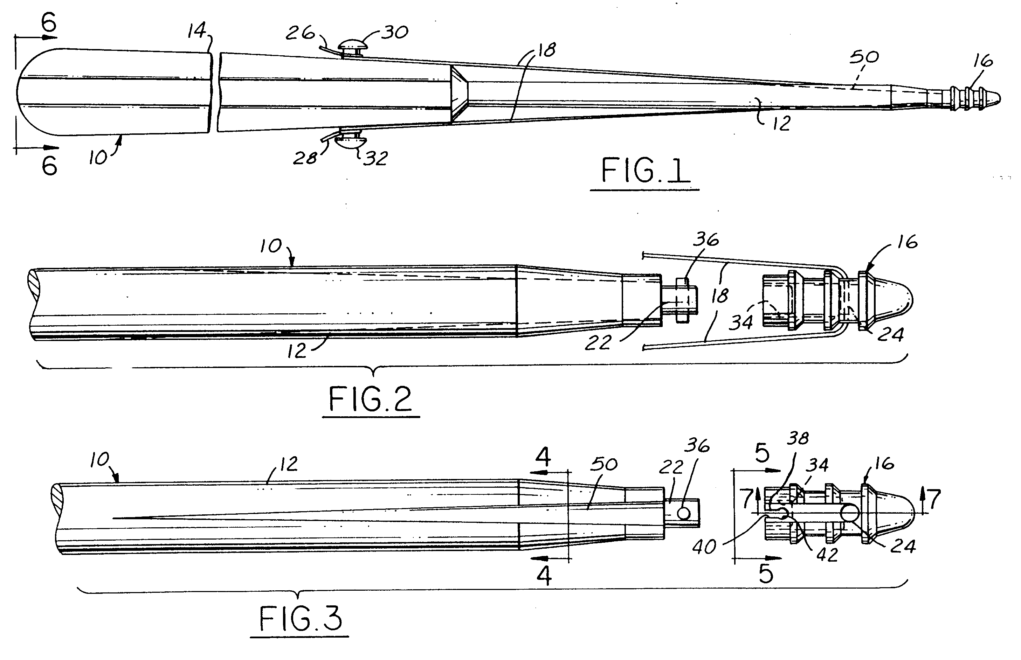

The features and details of the preferred embodiment of the unique anchor and driver assembly are shown in Figures 1-7. The driver device is generally designated by the reference numeral 10 and the anchor by the reference numeral 16.

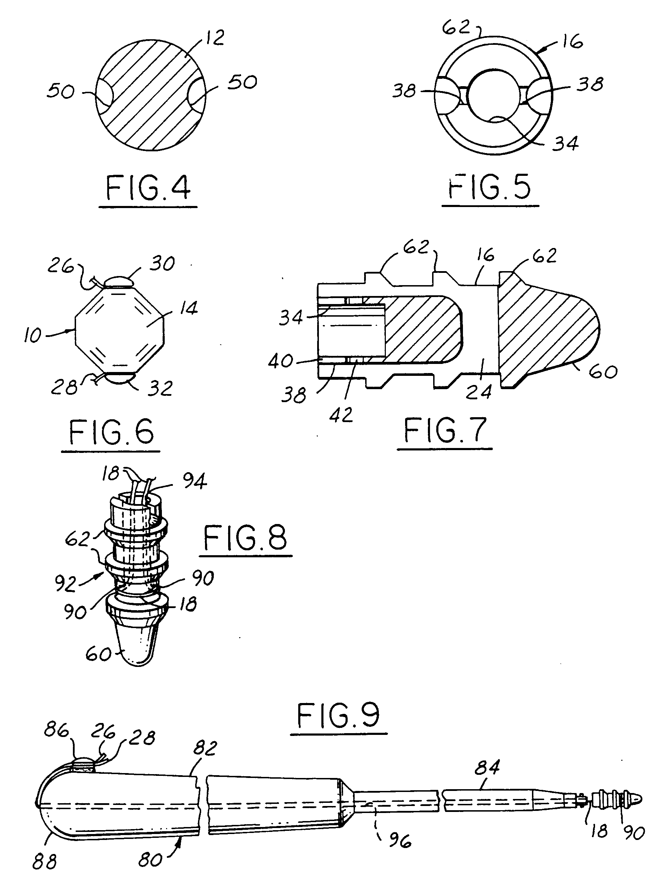

The driver device has a shaft member 12 attached or connected to a handle member 14. The shaft member and handle member can be separate members securely fastened together or they can be made from a single piece of material. Preferably the handle is a hollow stainless tube member having the shape shown in Figures 1 and 6 and the shaft is a stainless steel rod which is soldered, welded or otherwise affixed in or to the handle.

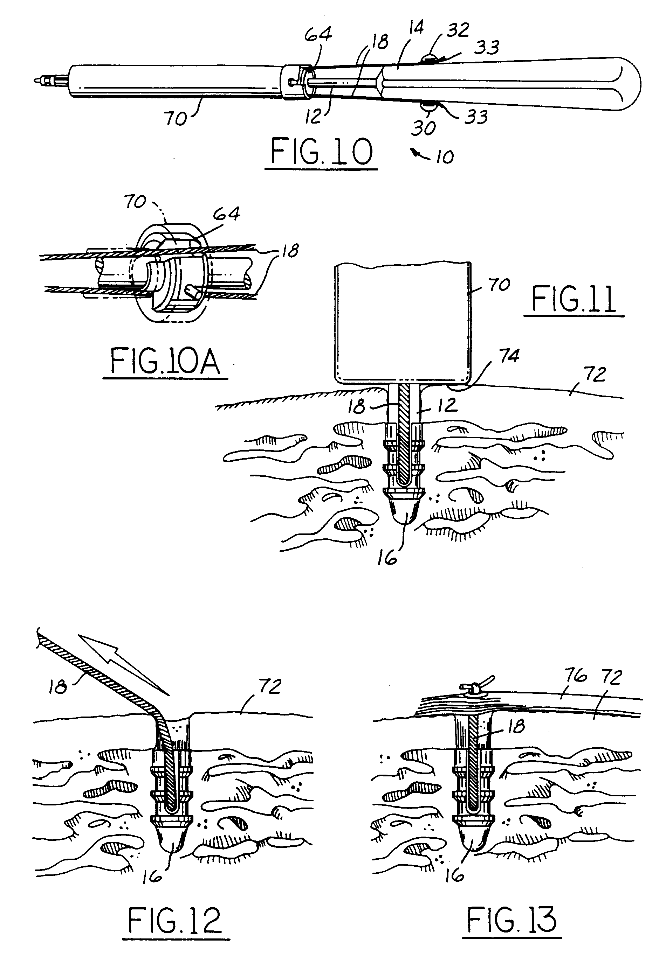

The anchor 16 is situated for installation (and also for removal if that is desired) on end projection 22 of the shaft 12, in a manner to be described in more detail below. A suture 18 is positioned through a hole or opening 24 in the anchor 16 and the two ends 26 and 28 of the suture are securely wrapped or tied around fixation posts 30 and 32, respectively. In order to securely hold the sutures which are wrapped on the posts 30 and 32, O-rings preferably are positioned on the stems of each of the posts. (O-rings 33 are shown in Figure 10.)

The positioning of the anchor 16 on end 22 and the affixation of the suture 18 on the fixation posts 30, 32 tightly and securely holds the anchor on the driver for use during surgery. In this manner, the anchor will not be subject to being dislodged, moved out of position on the shaft or accidently displaced from the shaft while the driver is being used to position and place the anchor during surgery. This is particularly important when the anchor is being used during arthroscopic knee, shoulder or other joint surgery and it would be harmful for the anchor to become loose in the joint.

The end projection 22 is adapted to fit within a mating recess or socket 34 in the anchor 16 (see Figures 2, 3 and 5). The cross-sectional size and shape of the projection 22 and socket 34 can be of any common geometric shape, but preferably have the same shape and are circular (as shown by Figure 5). If desired, the outside diameter of projection 22 and the inside diameter of socket 34 also can be dimensioned such that the two members have a frictional or slight force fit relationship.

A dowel or pin 36 is positioned in end projection 22 substantially perpendicular to the longitudinal axis of the driver device. The pin 36 projects on both sides of the end 22 forming two "ears" or "tabs".

The rod-type anchor 16 has a pair of slots 38 which are formed on opposite sides (180° apart) of the end of the anchor which fits on the driver device. The slots 38 are positioned and dimensioned to snap over and onto the protruding ends of the pin 36 on the driver. The slots 38 have an elongated passageway 40 which is slightly smaller in width than the cross-sectional diameter of the pin 36 and a larger generally circular end portion 42 which has substantially the same diameter as the pin 36.

The pin 36 "keys" the anchor to the shaft and driver so that the anchor cannot be rotated relative to the driver; the pin insures that the anchor can be rotated and maneuvered only with the driver.

The dimensioning of the slots 38 relative to the pin 36 also creates a force fit mating relationship and assembly between the anchor and driver device. The anchor 16 has to be forced onto the end 22 of the shaft by forcing the two ends of the pin 36 through the passageways 40 and into the openings 42. The anchor is resilient and "snaps" into place with the openings 42 on the shaft 36. This frictional or force-fit relationship also helps insure that the anchor and driver device will remain together during positioning and installation of the anchor during surgery.

The anchor is preferably made from Delrin 150SA® material, but it can be made of any other equivalent or compatible plastic or surgical material. It is also possible for some applications for the anchor to be made of a bio-absorbable material, such as polyglycolic acid (PGA) or polylactic acid (PLA).

The suture preferably utilized with the present invention is No. USP Size 2 Ticron material made by Davis & Geck Co. It is understood, of course, that any other equivalent suture or other material can be used so long as it satisfies the purposes and objects of the present invention.

The suture 18 can be placed through the hole in the anchor 16 before or after the anchor is positioned on the end of the driver shaft.

A pair of elongated slots or grooves 50 are provided on the shaft 12. (The grooves are best shown in Figures 3 and 4). The grooves are positioned 180° apart on the shaft and are of sufficient size and depth to position and retain the suture 18. The grooves 50 are oriented on the driver in axial alignment with the protruding ends of the pin 36 and with the fixation posts 30 and 32. When the anchor 16 is positioned on the driver 10, the suture 18 is tightly pulled into the grooves 50, down the length of the shaft 18 and securely wrapped and affixed around the posts 30 and 32. The ends 26 and 28 of the suture 18 are wound tightly around the fixation posts 30 and 32 which helps to hold the anchor on the driver during use.

After the anchor is positioned and installed in place, the ends of the suture are released from the posts 30 and 32 and the driver is pulled from the site. The fixation of the anchor 16 in the bone or tissue must be sufficiently strong and secure to allow the post 36 to be removed from the slots 38. Once the driver device is removed, the anchor 16 and suture 18 attached thereto are positioned in place for subsequent use during surgery. One such use is to fasten the end of a substitute or synthetic ACL ligament. In general, the anchor can be utilized to attach or reattach soft tissue, ligaments and tendons to bone. In these applications, the anchor would remain permanently in place. Another such use is the isometric testing of a proposed position for installation of an ACL replacement. In this application, the anchor and suture would be removed (and discarded) after the isometric testing was completed. In order to remove the anchor 16, the driver device is again utilized and the installation process reversed.

The shape and configuration of the preferred anchor 16 is best shown in Figures 2, 3, 5 and 7. The anchor has a front end or tip 60 which is rounded, conical or pointed for ease of placement and insertion. A sharp pointed anchor would allow it to pierce soft tissue more easily. A plurality of circular ridges 62 extend around the circumference of the anchor and are used to firmly and securely hold the anchor 16 in place in a hole.

When the hole for the anchor 16 is drilled or formed in the bone or tissue, the diameter of the drill should be slightly less than the outer diameter of the ridges 62. In this manner, the anchor 16 can be forced into position in the hole and the compression and friction caused by the ridges 62 against the inner walls of the hole will hold it firmly in place.

Preferably the outer diameter of the ridges is 4.29 mm (0.169 inches) and the length of the anchor is 10.4 mm (0.410 inches). The socket 34 has a diameter of 1.98 mm (0.078 inches) and a depth of 2.54 mm (0.100 inches). The diameter of the end projection 22 is preferably 1.93-1.95 mm (.076-.077 inches) and the length of the pin 36 is 2.46 mm (0.097 inches). The passageway of the slots 38 has a width of 0.63 mm (.025 inches) and the pin 36 has a diameter of 0.79 mm (0.0313 inches). The overall length of the driver device 10 (handle and shaft) is approximately 25.4 cm (10 inches). Other sizes and dimensions for the present invention can also be utilized.

Three ridges 62 are shown on the anchor 16 and utilized in the preferred embodiment for sufficient strength of the anchor and to provide the requisite holding force in the bone or tissue. It is understood, of course, that a greater or lesser number of ridges could be provided in accordance with the present invention so long as the objects and purposes of the present invention are obtained.

After the hole in the bone or tissue is formed (by drilling, for example with a K-wire having a diameter of 3.7 mm (0.146 inches)), the anchor 16 is "snapped" into position on the end 22 of the shaft of the driver and the suture 18 tightly wound on the fixation posts. The anchor and driver assembly is then maneuvered or moved into position by the surgeon (e.g. arthroscopically), and the tip 60 of the anchor positioned at or in the opening of the hole. The anchor 16 is then forced axially into the hole by, for example, the surgeon tapping on the end of the driver with a mallet or the like. It is not necessary to rotate the assembly in order to install it in position.

In order to avoid over-penetration of the anchor 16 into the bone, a guide member 70 is positioned over the shaft 12 of the driver instrument, as shown in Figure 10. The guide member is a hollow cylindrical member, preferably made of plastic, and slips over the driver and anchor assembly after the anchor is positioned on the driver device and the ends of the suture 18 are attached to the fixation posts 30 and 32. Preferably, the guide member 70 is held in place on the driver instrument 10 by a stop or collar member 64 which is soldered to the shaft 12 (see Figure 10A). The guide member 70 has a pair of opposed slots 66 which snap-fit over pins 68 on the stop member in order to hold it in place. The guide member also protects the sutures during use of the assembly during surgery. A series of guide members of different lengths can be provided in order to provide the insertion depth desired by the surgeon for the anchor. When the anchor is installed in the bone or bone-like structure 72, as shown in Figure 11, the end 74 of the guide member 70 contacts the bone and prevents the anchor from being inserted too far into the bone.

Once the anchor is firmly set in place, the ends 26 and 28 of the suture 18 are removed (unwound) from the fixation posts 30 and 32 and the driver is removed from the surgical site. Since the anchor is retained in the hole with a greater force than that required to snap and unsnap the anchor from the pin 36 on the driver, the driver can be removed easily from the anchor by the surgeon. The two ends of the suture 18 preferably are pulled firmly at an angle to the bone 72 (see Figure 12) to ensure a firm fit and more securely set the anchor in place. The anchor with suture attached is then used for its intended use. One such use is shown in Figure 13 where the suture 18 is tied through a soft tissue (ligament) 76 securely fastening it to the bone 72. In this manner, the ligament is mechanically reattached to the bone and, over time, the two members grow together and become permanently biologically reattached.

If the anchor is to be removed after use, or moved to another position, the installation process is repeated in reverse order, as mentioned earlier.

As an alternate embodiment, as shown in Figures 8 and 9, the driver device 80, consisting of handle 82 and shaft 84, is cannulated and the suture 18 is passed down the length of the driver and affixed on a fixation post 86 positioned adjacent the end 88 of the handle 82. The suture 18 is passed through opening 90 in anchor 92 and out through the open hollow interior 94 of the anchor. With this embodiment, a suture passer can be utilized to thread the suture through the passageway or channel 96 in the driver 80.

In order to gauge and measure the depth of insertion of the anchor member 110, markings or a scale 120 can be provided on the pusher member. The "hollow tube" driver device is particularly useful for insertion of small anchor member 110, such as on the order of about 1.59 mm (one-sixteenth of an inch) in diameter and 4.76 mm (three-sixteenths of an inch) in length.

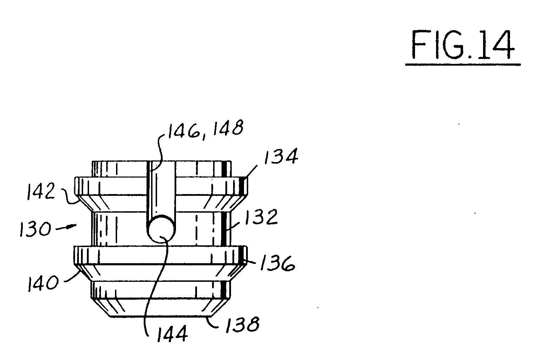

Another embodiment of the suture anchor is shown in Figure 14. The anchor 130 has a rod-shaped body 132 with a pair of annular rings 134 and 136 on its outer surface. The front end 138 of the anchor is flat with a 45° chamfer around the edge in order to ease entry of the anchor into the hole in the bone. Chamfer or angled surfaces 140 and 142 are provided on the leading edges of the rings 134 and 136 also in order to ease entry of the anchor into the bone. A hole 144 with opposed slots 146 and 148 are provided for positioning of the suture.

## Claims

1. A device for anchoring a suture to a bone, said device comprising:
a driver (10) having a handle (14) and an elongated shaft (12), said shaft having two ends, one of said ends attached to said handle;
an anchor member (16) having a hollow chamber (34) at one end for receipt of said other of said ends of said shaft in a mating relationship;
at least one end projection (22) positioned proximate to the other of said ends of said shaft (12);
said anchor member (16) having at least one surface opening into said hollow chamber (34) for receipt of said end projection (22) when said anchor member is positioned on said shaft;
**characterized** in that said anchor member (16) defines means (24) for receiving a portion of the suture (18) through at least one wall of said anchor member; and
attachment means are provided on said handle (14) for securing another portion of the suture thereto to maintain interconnection between said anchor member (16) and said driver (10),
wherein said end projection (22) includes at least two tab members (36) each of which extends radially outwardly from said shaft (12) in a direction substantially normal to the longitudinal axis of said shaft.

2. The device of Claim 1 wherein said attachment means includes at least two posts (30, 32) disposed on substantially opposite sides of said handle, each post securing a different portion of the suture to apply tension upon said anchor member.

3. The device of Claim 1 further comprising ridge means (62) on said anchor member (16) for assisting in the securing of said anchor member in said bone.

4. The device of Claim 1 further comprising elongated channel means on the exterior surface of said shaft (12) for placement of a portion of the suture extending between said anchor member (16) and said handle (14).

5. The device of Claim 4 wherein said channel means comprises a pair of opposed channels (50) on said shaft (12).

6. The device as set forth in Claim 1 wherein said means for receiving is a passageway (24) in said anchor member (16) passing through opposing walls of said anchor member, said passageway being substantially normal to the longitudinal axis of said anchor member.

7. The device of Claim 1 further comprising a guide member for positioning over said shaft member and limiting the depth of insertion of said anchor member.

8. The device of Claim 7 wherein said guide member comprises a hollow cylinder.

## Patentansprüche

1. Vorrichtung zum Verankern eines Nähfadens an einem Knochen, wobei die Vorrichtung folgendes umfaßt:
einen Treiber (10) mit einem Handgriff (14) und einem länglichen Schaft (12) wobei der Schaft zwei Enden aufweist, von denen das eine Ende an dem Handgriff befestigt ist;
ein Verankerungsorgan (16), das an seinem einen Ende eine hohle Kammer (34) aufweist zur passenden Aufnahme des anderen der erwähnten Enden des Schafts;
mindestens einen Endvorsprung (22), der in der Nähe des anderen der erwähnten Enden des Schafts (12) positioniert ist
wobei das Verankerungsorgan (16) mindestens eine Oberflächenöffnung in der erwähnten hohen Kammer (34) aufweist zur Aufnahme des erwähnten Endvorsprungs (22) wenn das Verankerungsorgan auf dem Schaft positioniert ist;
dadurch gekennzeichnet, daß das Verankerungsorgan (16) Einrichtungen (24) begrenzt für die Aufnahme eines Abschnitts des Nähfadens (18) durch mindestens eine Wand des Verankerungsorgans;
Befestigungseinrichtungen auf dem Handgriff (14) vorgesehen sind, um einen anderen Abschnitt des Nähfadens daran festzulegen, um die Verbindung zwischen dem Verankerungsorgan (16) und dem Treiber (10) aufrechtzuerhalten,
wobei der Endvorsprung (22) mindestens zwei Nasen (36) umfaßt, die sich jeweils radial von dem Schaft (12) nach außen erstrecken in eine Richtung, die im wesentlichen senkrecht zu der Längsachse des Schafts steht.

2. Vorrichtung gemäß Anspruch 1, wobei die Befestigungseinrichtung mindestens zwei Pfosten (30,32) umfaßt, die an im wesentlichen gegenüberliegenden Seiten des Handgriffs angeordnet sind, wobei an jedem Pfosten ein anderer Abschnitt des Nähfadens so befestigt ist, daß das Verankerungsorgan mit Zugspannung beaufschlagt wird.

3. Vorrichtung gemäß Anspruch 1, ferner umfassend Riffeleinrichtungen (62) auf dem Verankerungsorgan (16) um die Festlegung des Verankerungsorgans in dem Knochen zu unterstützen.

4. Vorrichtung gemäß Anspruch 1, ferner umfassend längliche Kanaleinrichtungen an der äußeren Oberfläche des Schafts (12) für die Platzierung eines Abschnitts des Nähfadens, der sich zwischen dem Verankerungsorgan (16) und dem Handgriff (14) erstreckt.

5. Vorrichtung gemäß Anspruch 4, wobei die erwähnten Kanaleinrichtungen ein paar von gegenüberliegenden Kanälen (50) auf dem Schaft (12) umfassen.

6. Vorrichtung gemäß Anspruch 1, wobei die Einrichtung zur Aufnahme ein Durchgang (24) in dem Verankerungsorgan (16) ist, der durch gegenüberliegende Wände des Verankerungsorgans hindurchgeht, wobei der Durchgang im wesentlichen senkrecht zur Längsachse des Verankerungsorgans verläuft.

7. Vorrichtung gemäß Anspruch 1, ferner umfassend ein Führungsorgan, das über das Schaftorgan positioniert werden kann und die Eindringtiefe des Verankerungsorgans begrenzt.

8. Vorrichtung gemäß Anspruch 7, wobei das Führungsorgan einen hohlen Zylinder umfaßt.

## Revendications

1. Dispositif pour ancrer une suture à un os, ledit dispositif comprenant :
- un organe d'entraînement (10) ayant un manche (14) et une tige allongée (12), ladite tige ayant deux extrémités, l'une desdites extrémités étant fixée audit manche ;
- un élément d'ancrage (16) ayant une chambre creuse (34) à l'une de ses extrémités pour recevoir ladite autre desdites extrémités de ladite tige dans une relation d'accouplement ;
- au moins un prolongement d'extrémité (22) positionné à proximité de l'autre desdites extrémités de ladite tige (12) ;
- ledit élément d'ancrage (16) ayant au moins une surface s'ouvrant dans ladite chambre creuse (34) pour recevoir ledit prolongement d'extrémité (22) lorsque ledit élément d'ancrage est positionné sur ladite tige ;
caractérisé par le fait que ledit élément d'ancrage (16) définit des moyens (24) pour la réception d'une partie de la suture (18) à travers au moins une paroi dudit élément d'ancrage ; et
des moyens de fixation sont disposés sur ledit manche (14) pour fixer une autre partie de la suture à celui-ci, afin de maintenir une interconnexion entre ledit élément d'ancrage (16) et ledit organe d'entraînement (10),
ledit prolongement d'extrémité (22) comprenant au moins deux pattes (36) dont chacune s'étend radialement vers l'extérieur à partir de ladite tige (12), dans une direction sensiblement perpendiculaire à l'axe longitudinal de ladite tige.

2. Dispositif selon la revendication 1, dans lequel lesdits moyens de fixation comprennent au moins deux supports (30, 32) disposés sur des côtés sensiblement opposés dudit manche, chaque support assurant la fixation d'une partie différente de la suture pour appliquer une tension sur ledit élément d'ancrage.

3. Dispositif selon la revendication 1, comprenant en outre des moyens en forme d'arête (62) situés sur ledit élément d'ancrage (16) pour aider à la fixation dudit élément d'encrage dans ledit os.

4. Dispositif selon la revendication 1, comprenant en outre des moyens en forme de canal allongé, situés sur la surface extérieure de ladite tige (12) afin de mettre en place une partie de la suture s'étendant entre ledit élément d'ancrage (16) et ledit manche (14).

5. Dispositif selon la revendication 4, dans lequel lesdits moyens en forme de canal comprennent deux canaux opposés (50) ménagés sur ladite tige (12).

6. Dispositif selon la revendication 1, dans lequel lesdits moyens de réception sont un passage (24) ménagé dans ledit élément d'ancrage (16) et passant à travers des parois opposées dudit élément d'ancrage, ledit passage étant sensiblement perpendiculaire à l'axe longitudinal dudit élément d'ancrage.

7. Dispositif selon la revendication 1, comprenant en outre un élément de guidage destiné à être positionné sur ladite tige et à limiter la profondeur d'insertion dudit élément d'ancrage.

8. Dispositif selon la revendication 7, dans lequel ledit élément de guidage comprend un cylindre creux.
